# EUROPEAN PATENT APPLICATION

(11) **EP 3 839 909 A1**
(43) Date of publication of application: **23.06.2021**
(21) Application number: 19217294.8
(22) Date of filing: 18.12.2019
(51) Int. Cl.: G08B 13/196, G08B 13/16, G08B 21/04, A61B 5/00, H04N 7/18

(54) **DETECTING THE PRESENCE OF AN OBJECT IN A MONITORED ENVIRONMENT**

(71) Applicant: Koninklijke Philips N.V., 5656 AG Eindhoven (NL)
(72) Inventor: MATTHEIJ, Rudolphus Johannes Hubertus, 5656 AE Eindhoven (NL)
(74) Representative: Philips Intellectual Property & Standards

(57) **Abstract**

Proposed are concepts for determining the presence of an object (e.g. a monitored subject or person, or an object carried by a monitored subject/person) in an environment (such as a room) even when the object is not within the field of view of a video capture system that is adapted to monitor the environment. Such concepts may be based on detecting a sound with an audio characteristic which matches that associated with an object. It is proposed that, if this occurs when the object is not within the field of view of the video system, it can be inferred that the object is within the monitored environment but not within the field of view.

## Description

### FIELD OF THE INVENTION

The invention relates to monitoring an environment, and more particularly to detecting the presence of an object in a monitored environment.

### BACKGROUND OF THE INVENTION

It is known to monitor an environment with a video capture system and, recently, 'smart' video capture systems have been developed which can track the location of an object (e.g. a subject or person) within a monitored environment. Such systems may therefore be employed where automatic and/or continuous monitoring is required, for example in the field of automatic subject (e.g. patient or prisoner) monitoring.

However, due to spatial constraints (e.g. a limited number of locations where a video camera can be mounted safely and/or securely), it may not be possible or practical to arrange the Field of View (FOV) of a video capture system to cover the entirety of the monitored environment. In other words, the FOV of a video camera system may be such that one or more parts of the monitored environment are outside of the FOV. In such situations, the video capture system is not able to detect and monitor objects within the part(s) of the monitored environment outside the FOV of the video capture system.

### SUMMARY OF THE INVENTION

The invention is defined by the claims.

According to examples in accordance with an aspect of the invention, there is provided a method for detecting the presence of an object in a monitored environment, the monitored environment being monitored using an audio capture system adapted to detect sound in the monitored environment and a video capture system having a field of view that does not cover the entirety of the monitored environment, the method comprising: detecting, based on an audio signal from the audio capture system, an indicative sound having at least one audio characteristic matching an audio characteristic associated with an object; determining, based on a video signal from the video capture system, that the object is not present in the field of view, the video signal relating to a time period during which the indicative sound occurred; and responsive to: detecting the indicative sound; and determining the object is not present in the field of view, determining that the object is present in the monitored environment.

Proposed are concepts for determining the presence of an object (e.g. a monitored subject or person, or an object carried by a monitored subject/person) in an environment (such as a room) even when the object is not within the field of view of a video capture system that is adapted to monitor the environment. Such concepts may be based on detecting a sound with an audio characteristic that matches that associated with an object. It is proposed that, if this occurs when the object is not within the field of view of the video system, it can be inferred that the object is within the monitored environment but not within the field of view (i.e. present, but outside the FOV).

Embodiments may leverage a proposed concept that specific audio characteristics can be uniquely associated with objects (such as a subject, or an object carried or worn by a subject). By detecting a sound with an audio characteristic, an object associated with that characteristic may be identified. If it is then determined that the object is not present within concurrently captured video (i.e. video captured for the same period of time during within which the sound occurred), it may be concluded that the object is present within the environment monitored by the audio capture system (but not within the field of view of the video capture system). Such a conclusion may be validated or confirmed based on the video captured before (and/or after) the occurrence of the sound. For example by identifying the presence of the object within earlier (and/or later) captured video, it may confirm that the object was present within the field of view of the video capture system before and/or after the occurrence of the sound.

Proposed embodiments may therefore facilitate a capability of detecting and monitoring an object outside of the FOV of a video capture system. Improved and more robust object detection, tracking and monitoring may thus be facilitated by embodiments.

According to some embodiments, audio characteristics may have already been associated with objects. Such embodiments therefore need not include a process of correlating sounds to objects within the FOV of the video system. In particular, the inventors have realized that prior information about objects and their associated sound characteristics may be leveraged (e.g. imported from another source) so that no such correlations and assignment of audio identifiers needs to be undertaken by perceived implementations. Rather, proposed embodiments may be configured to simply detect sound having certain characteristics that match those which have been predetermined elsewhere and assigned to an object (e.g. as an audio profile). This approach still enables an embodiment to distinguish between sounds emitted from a monitored subject and, say, a door, whilst avoiding the need to correlate (e.g. bind/associate an audio profile using) a detected audio location and an object detected in a video.

Other embodiments may, however, include additional steps of correlating the locations of detected sounds with locations of objects in captured video, and then assigning characteristics of the sound(s) to the corresponding objects (e.g. by creating audio profiles and associating the audio profiles with objects). Put another way, some embodiments may include a concept of determining one or more audio characteristics and associating the audio characteristic(s) with an object detected in captured video. This may provide the advantage that it allows the association (i.e., binding) of descriptive audio signals emitted by an object (e.g. subject) to that object. In this way, embodiments may distinguish between the sounds that are made by that object and sounds from another object of no interest (e.g. a squeaking door). Once an audio source is bound to a specific object, embodiments may enable tracking of the object well beyond the field of view of the camera.

There may be proposed a system that analyses an object's (e.g. subject's) descriptive audio characteristics when in the FOV of the video capture system. When the object then moves outside of the FOV of the video capture system, the system may be able to locate and track the position of that object by correlating detected sounds outside with the object's descriptive audio characteristics. Providing an ability to detect and track an object outside of the FOV may provide for more robust object localization and/or tracking.

Some embodiments may further comprise determining an identifier of the object based on the at least one audio characteristic of the indicative sound. Further, determining an identifier of the object may comprise comparing the at least one audio characteristic with a set of audio identifiers associated with a plurality of different objects, each audio identifier being associated with a respective object and comprising one or more audio characteristics associated with the respective object. Proposed embodiments may thus include a process of identifying one of a set of potential object based on the audio characteristics of the indicative sound. Put another way, it may be identified which audio profile matches the detected indicative sound in order to determine an object producing the sound. Thus, simple matching processes and/or techniques may be employed by embodiments, thereby reducing implementation complexity and/or cost.

Proposed embodiments may also include the step of estimating the location of the object within the monitored environment based on a historical video signal from the video capture system, the historical video signal relating to an earlier time period prior to the occurrence of the indicative sound and during which the object was present in the field of view. By way of example, estimating the location of the object within the monitored environment may comprise: analyzing the historical video signal to determining at least one of a location and direction of travel of the object in the field of view; and extrapolating from the determined location and/or direction of travel of the object in the field of view to estimate the location of the object. In this way, a previous location and/or movement of the object in captured video may be used to determine where the object may be outside of the FOV, e.g. by extrapolation.

Some embodiments may further comprise: determining, using an audio location system, a first location of source of a sound detected by the audio capture system; determining, based on a video signal from the video capture system, a second location of an object present in the field of view; determining if the first and second locations match; and, responsive to determining that the first and second locations match, associating at least one audio characteristic of the detected sound with the object. For instance, associating at least one audio characteristic of the detected sound with the object may comprise: analyzing the detected sound to determine one or more audio characteristics of the detected sound; generating an audio identifier comprising information relating to the determined one or more audio characteristics of the detected sound; and associating the generated audio identifier with the object. In this way, embodiments may employ a concept of correlating the locations of detected sounds with locations of objects in captured video, and then assigning characteristics of the sound(s) to the corresponding objects (e.g. by creating audio profiles and associating the audio profiles with objects.

The at least one audio characteristic comprises one or more of: pitch; loudness; timbre or tone color; repetitive pattern; distinctive sound; and duration. In this way, various different characteristics of the sound may be employed for object identification purposes.

Purely by way of example, the object may comprise a subject (e.g. a patient) and the monitored environment may comprise a room of a building (such as hospital ward, hospital room, bedroom, etc.). Embodiments may therefore be used to monitor a subject (e.g. patient) within a hospital room for example. Illustrative embodiments may be utilized in many different types of clinical, medical or patient-related environments, such as a hospital, doctor's office, ward, care home, person's home, etc.

According to another aspect, there is provided a computer program product for detecting the presence of an object in a monitored environment, the monitored environment being monitored using an audio capture system adapted to detect sound in the monitored environment and a video capture system having a field of view that does not cover the entirety of the monitored environment, wherein the computer program product comprises a computer-readable storage medium having computer-readable program code embodied therewith, the computer-readable program code configured to perform all of the steps of a proposed embodiment.

Thus, there may also be provided a computer system comprising: a computer program product according to proposed embodiment; and one or more processors adapted to perform a method according to a proposed concept by execution of the computer-readable program code of said computer program product.

According to still another aspect of the invention, there is provided a system for detecting the presence of an object in a monitored environment, the monitored environment being monitored using an audio capture system adapted to detect sound in the monitored environment and a video capture system having a field of view that does not cover the entirety of the monitored environment, the system comprising: an audio analysis unit configured to detect, based on an audio signal from the audio capture system, an indicative sound having at least one audio characteristic matching an audio characteristic associated with an object; a video analysis unit configured to determine, based on a video signal from the video capture system, that the object is not present in the field of view, the video signal relating to a time period during which the indicative sound occurred; and a decision unit configured, responsive to: the audio analysis unit detecting the indicative sound; and the video analysis unit determining the object is not present in the field of view, to determine that that the object is present in the monitored environment.

The system may be remotely located from a user device. In this way, a user (such as a medical professional) may have an appropriately arranged system that can receive information at a location remotely located from the system for automatic and dynamic detection of the presence of an object in a monitored environment. Embodiments may therefore enable a user to monitor an environment using a local system (which may, for example, comprise a portable display device, such as a laptop, tablet computer, mobile phone, PDA, etc.). By way of example, embodiments may provide an application for a mobile computing device, and the application may be executed and/or controlled by a user of the mobile computing device.

The system may further include: a server device comprising the system for detecting the presence of an object in a monitored environment; and a client device comprising a user-interface. Dedicated data processing means may therefore be employed for the purpose of detecting the presence of an object in a monitored environment, thus reducing processing requirements or capabilities of other components or devices of the system.

The system may further include a client device, wherein the client device comprises the decision unit and a display unit. In other words, a user (such as a doctor, caregiver or medical professional) may have an appropriately arranged client device (such as a laptop, tablet computer, mobile phone, PDA, etc.) which processes received data in order to determine that an object is present in the monitored environment and generate a display control signal. Purely by way of example, embodiments may therefore provide a monitoring or observation system that enables monitoring of one or more environments (each including subjects or patients for example) from a single location, wherein real-time communication between a monitored environment and monitoring user (e.g. nurse or doctor) is provided and can have its functionality extended or modified according to proposed concepts, for example.

It will be understood that processing capabilities may therefore be distributed throughout the system in different ways according to predetermined constraints and/or availability of processing resources.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for detecting the presence of an object in a monitored environment according to an embodiment;
Fig. 2 depicts a simplified block diagram of system for detecting the presence of an object in a monitored environment according to an embodiment; and
Figs. 3A-3C illustrate an exemplary embodiment employing a modified version of the system of Fig. 2.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

For example, reference to video capture system should not be taken as being limited to conventional color (e.g. RGB) or visual data camera-based systems, but should instead be understood to other forms of camera-based systems that have a field-of-view, such as capture systems that employ stereo cameras or 3D/depth/time-of-flight cameras.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Proposed is an approach for enabling the presence of an object in a monitored environment even when the object is outside the FOV of a video capture system configured to monitor the environment. Embodiments may detect an indicative sound having an audio characteristics associated with the object, and then, if that object is not present in the FOV of simultaneously captured video of the monitored environment, it can be determined that the object is present in the monitored environment.

Such proposals may thus facilitate simple and intuitive detection of an object in a monitored environment by leveraging known (or determined) audio characteristics of the object, and this may for example be done in consideration of a context of captured video of the environment.

Embodiments of the present invention are therefore directed toward monitoring one or more objects within an environment, and aim to overcome the limitation that a video capture system may not have a FOV that does not entirely cover the environment.

By way of example only, illustrative embodiments may be utilized in many different types of clinical, medical or subject-related environments, such as a hospital, doctor's office, ward, care home, person's home, etc. For instance, embodiments may be employed to monitor a patient in a hospital room. Other embodiments may be used to monitor a prisoner within a prison cell.

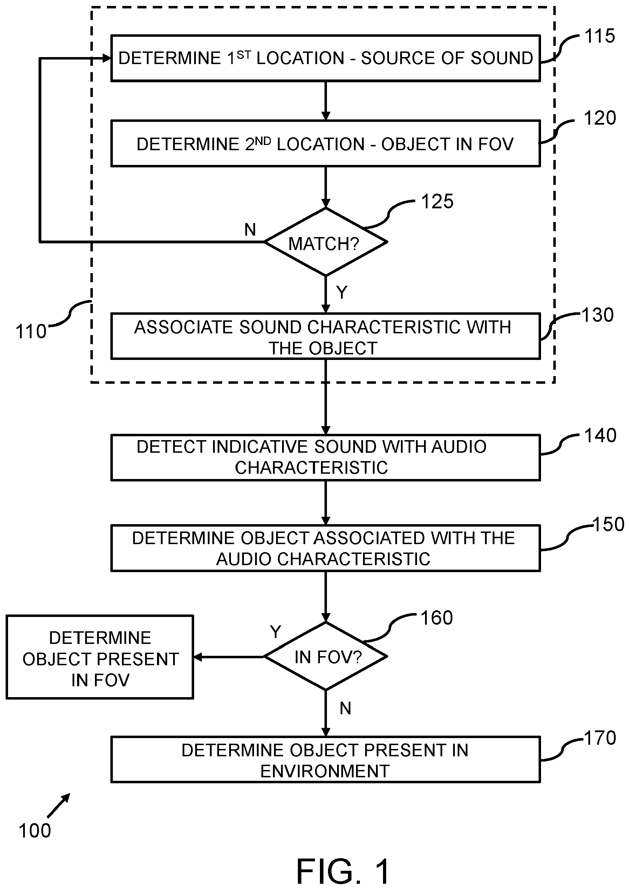

Fig. 1 is a simplified flow diagram of a method for detecting the presence of an object in a monitored environment according to an embodiment. For the purpose of describing this embodiment, the monitored environment is monitored using an audio capture system that is adapted to detect sound in the monitored environment. The monitored environment is also monitored using a video capture system having a FOV that does not cover the entirety of the monitored environment (i.e. the monitored environment comprises one or more sections/areas that are not within the FOV of the video capture system). By way of the example, the monitored environment of this embodiment is a room of a building, specifically a hospital room. Also, the object is a human subject, namely a patient admitted to the hospital.

The method begins with the process 110 of correlating a location of a detected sound with a location of an object in video captured by the video capture system, and then assigning a characteristics of the sound to the corresponding object. Put another way, the method begins with a process 110 of determining an audio characteristic of a detected sound and associating the audio characteristic with an object detected in captured video.

Specifically, the process 110 comprises steps 115 to 130. Step 115 comprises determining, using an audio location system (such as a conventional beam forming microphone apparatus), a first location of source of a sound detected by the audio capture system. Then, step 120 comprises determining, based on a video signal from the video capture system, a second location of an object present in the FOV of the video capture system. Next, in step 125, it is determined if the first and second locations match (i.e. are substantially equal or coincide).

If it is determined in step 125 that the first and second locations match, it is inferred that the source of the detected sound is the object. The method therefore proceeds to step 130, wherein at least one audio characteristic of the detected sound is determined and then associated with the object (e.g. assigned to an audio profile linked to the object). Specifically, in this example, step 130 of associating at least one audio characteristic of the detected sound with the object comprises: analyzing the detected sound to determine one or more audio characteristics of the detected sound; generating an audio identifier comprising information relating to the determined one or more audio characteristics of the detected sound; and associating the generated audio identifier with the object.

Conversely, if it is determined in step 125 that the first and second locations do not match, it is inferred that the source of the detected sound is not the object. The method then returns to step 115 to repeat the process of correlating a location of a detected sound with a location of an object in video captured by the video capture system.

After completing the process 110 of associating audio characteristics with objects detected in captured video, the method proceeds to step 140.

Step 140 comprises detecting, based on an audio signal from the audio capture system, an indicative sound having an audio characteristic matching an audio characteristic associated with an object. By way of example, an audio characteristic may comprise: pitch; loudness; timbre or tone color; repetitive pattern; distinctive sound; and duration.

Based on the audio characteristic of the indicative sound, an identifier of the object is determined in step 150. Specifically, step 150 comprises comparing the audio characteristic with a set of audio identifiers associated with a plurality of different objects, each audio identifier being associated with a respective object and comprising one or more audio characteristics associated with the respective object. Put another way, step 150 comprises determine an object that is associated with the audio characteristic of the indicative sound.

After identifying the object associated audio characteristic of the indicative sound (i.e. after completing step 150), the method proceeds to step 160. In step 160, it is determined, based on a video signal from the video capture system, whether not the object is present in the field of view. For the avoidance of doubt, it is noted that the video signal assessed relate to a time period during which the indicative sound occurred. In other words, it is checked whether the indicative sound occurred simultaneously with the object being in the FOV of the video capture system.

If it is determined in step 160 that the object is not present in the FOV, it is inferred that the object is within the monitored environment (because it is a source of sound detected within the monitored environment) but not within the FOV of the video capture system. Thus, the method proceeds to step 170 wherein it is determined that the object is present in the monitored environment.

Conversely, if it is determined in step 170 that the object is present in the FOV, the method proceeds to step 170 wherein it is determined that the object is present in both the monitored environment and the FOV of the video capture system.

Although not depicted in the embodiment of Fig. 1, it is noted that some embodiments may also include a process of estimating the location of the object within the monitored environment based on a historical video signal from the video capture system. For instance, the historical video signal may relate to an earlier time period prior the occurrence of the indicative sound and during which the object was present in the field of view. By way of example, the historical video signal may be analyzed to determine a location and/or direction of travel of the object in the FOV. By extrapolating from the determined location and/or direction of travel of the object in the FOV, the location of the object may be estimated.

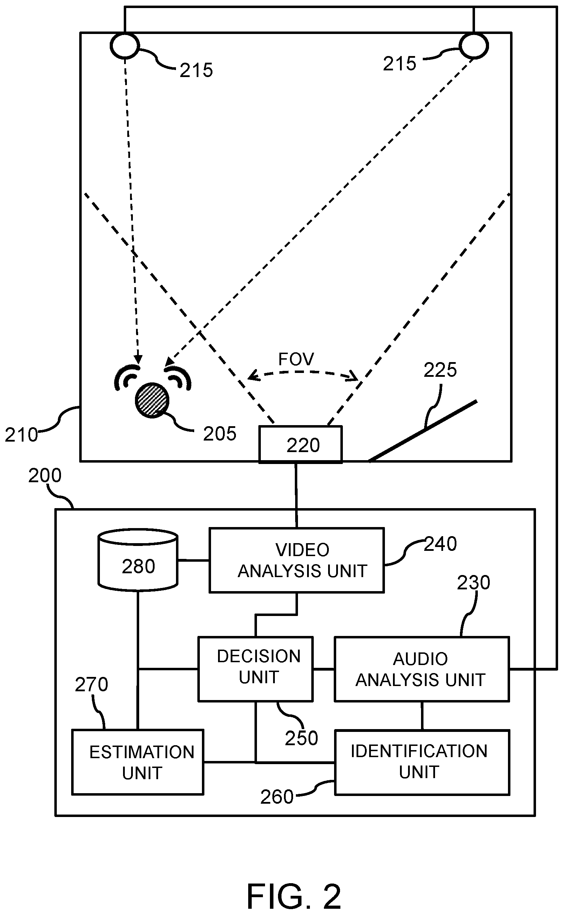

By way of further illustration of the proposed concept(s), a system for detecting the presence of an object in a monitored environment according to an embodiment will be now be described with reference to Fig. 2.

Fig. 2 depicts a simplified block diagram of system 200 for detecting the presence of an object 205 in a monitored environment according to an embodiment. Fig. 2 also depicts the monitored environment 210 which is monitored using an audio capture system 215 (comprising a plurality of microphones 215) adapted to detect sound in the monitored environment 210 and a video capture system 220 having a field of view FOV that does not cover the entirety of the monitored environment 210. Here, the object 205 is a subject (i.e. person) and the monitored environment comprises a room of a building, the room having a door 225.

The system 200 comprises an audio analysis unit 230 configured to detect, based on an audio signal from the audio capture system 215, an indicative sound having at least one audio characteristic matching an audio characteristic associated with an object 205.

The system 200 also comprises a video analysis unit 240 configured to determine, based on a video signal from the video capture system 220, that the object 205 is not present in the field of view FOV, the video signal relating to a time period during which the indicative sound occurred.

The system 200 yet further includes a decision unit 250 (e.g. a processor) configured, responsive to: the audio analysis unit 230 detecting the indicative sound; and the video analysis unit 240 determining the object is not present in the field of view, to determine that the object is present in the monitored environment 210.

In this embodiment, the system 200 also includes an identification unit 260 that is configured to determine an identifier of the object based on the at least one audio characteristic of the indicative sound. Yet further, the system 200 also includes an estimation unit 270 that is configured to estimate the location of the object 205 within the monitored environment 210 based on a historical video signal from the video capture system. Here, the historical video signal relates to an earlier time period prior to the occurrence of the indicative sound and during which the object was present in the field of view. Such historical video signals are stored in a data store 280 of the system.

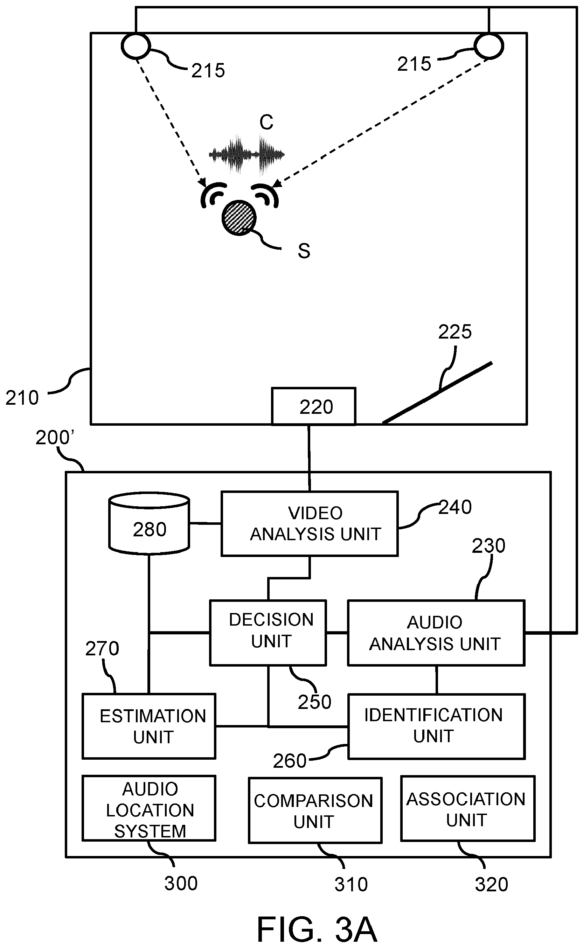

By way of further illustration of the proposed concept(s), an exemplary embodiment will be now be described with reference to Figs. 3A-3C. Here, it is noted that the system 200' of Figs. 3A-3C is a modified version of the system 200 of Fig. 2. Specifically, the system 200' in Figs. 3A-3C is similar to that of Fig. 2 but further comprises an audio location system 300, a comparison unit 310, and an association unit 320.

The audio location system 300 is configured to determine a first location of source of a sound detected by the audio capture system, and to determine, based on a video signal from the video capture system, a second location of an object present in the field of view. The comparison unit 310 is configured to determine if the first and second locations match, and the association unit 320 is configured, responsive to determining that the first and second locations match, to associate at least one audio characteristic of the detected sound with the object.

Referring now to Fig. 3A, the audio capture system 215 captures all audio signals in the monitored environment 210. The audio location system 300 analyses the audio signals from the audio capture system 215 to identify the location of a source S of sound detected by the audio capture system 215. Here, the detected sound has a characteristic C that is determined by the audio analysis unit.

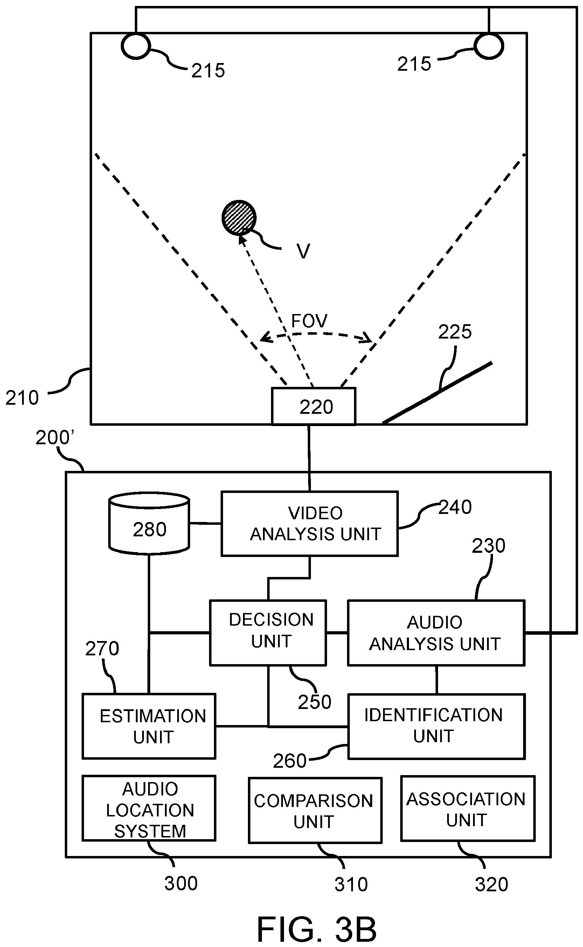

Referring now to Fig. 3B, the video capture system 220 captures all video signals covering the field of view FOV within the monitored environment 210. The audio location system 300 analyses the video signals from the video capture system 220 to identify the location of an object V visually present in the video signals from the video capture system 220. Here, the object V is the source of the sound S having audio characteristic C that was detected in Fig. 3A.

The comparison unit 310 compares the detected location of the source S of sound with the location of the object V visually present in the video signals, so as to determine if the location of the source of sound coincides (i.e. matches) the location of the object V in the video signals. In this way, the system 200' correlates the location of the object V within the field of view FOV with the location of the source S of sound. The association unit 320 associates the audio characteristic C of the detected sound with the object. By way of example, the association unit 230 assigns a unique audio identifier (i.e., a profile describing the characteristic audio signals origination from that particular source) to the detected object.

The system 200' (visually) tracks the location of the object 205 within the field of view FOV of the video capture system.

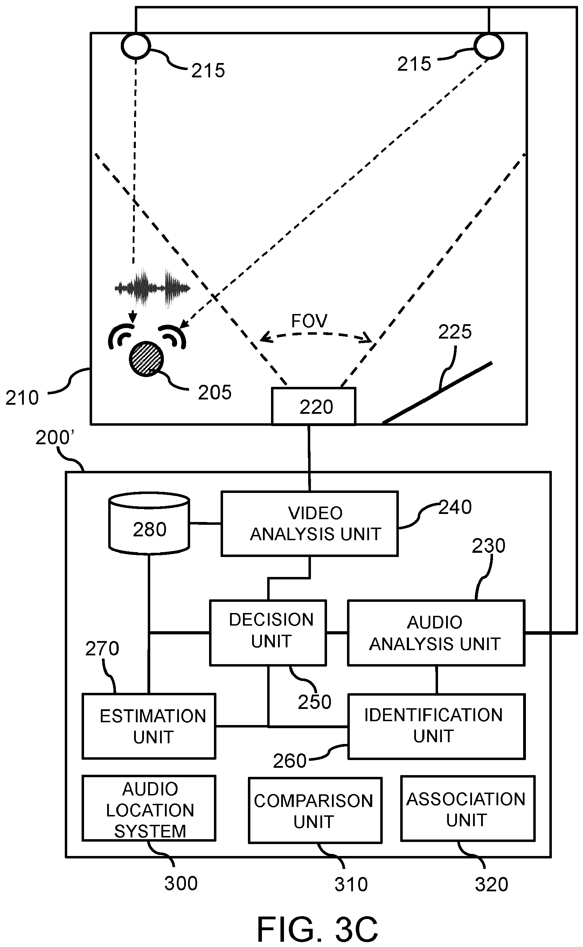

When the object 205 moves out of the field of view FOV, as depicted in Fig. 3C, the system 200' is able to localize the object 205 within the monitored environment 210 by correlating the object's unique audio identifier with the signals of an audio source detected outside the field of view FOV.

To aid understanding of the proposed concept(s), one may now consider an example comprising: (i) a smart camera that is mounted high on a wall within a prison cell; and, (ii) one or more audio capture devices that are positioned in the prison cell. An exemplary method according to embodiment may then be summarized as follows:
STEP 1: The system uses the smart camera in the prison to recognize a subject (e.g. prisoner) standing and walking inside the FOV of the camera. Upon detection of a subject within the FOV, the system tracks the subject's location.
STEP 2: The system uses the audio capture devices to capture all audio signals in the prison cell. The system localizes the source(s) of the captured audio signal(s).
STEP 3: The system then correlates the location of the subject within the FOV (as captured with the smart camera) with the location of any of the source(s) of audio signal(s) at that location.
STEP 4: If the locations match (i.e., the source of the audio signals originates from the same location where the smart camera detects a subject), the system uses AI or Machine Learning techniques to perform an additional analysis and learn any distinctive audio characteristics emitted by each audio source, such as, but not limited to: vocal characteristics (e.g., volume, pitch, rate, pauses, vocal variety, pronunciation, articulation, ...) and/or non-vocal characteristics (e.g., sounding footsteps, moving of clothing, grinding sounds, ...).
STEP 5: The system assigns a unique audio identifier (i.e., a profile describing the characteristic audio signals origination from that particular source) to the detected subject. In this way, a unique audio profile of the subject is defined, which, in turn, allows recognition of a subject person by correlating the subject's unique audio profile with the signals of a given audio source.
STEP 6: The system (visually) keeps track of the location of the subject within the FOV of view of the smart camera. When the subject moves out the FOV of the camera, the system is still able to localize the subject within the prison cell by correlating the subject's unique audio identifier with the signals of a detected audio source outside the FOV of the camera.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

From the above-describe embodiments, it will be understood that there is proposed a concept of audio-assisted field-of-view extension that may be used for subject detection and monitoring within a monitored environment.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A method for detecting the presence of an object in a monitored environment, the monitored environment being monitored using an audio capture system adapted to detect sound in the monitored environment and a video capture system having a field of view that does not cover the entirety of the monitored environment, the method comprising:
detecting (140), based on an audio signal from the audio capture system, an indicative sound having at least one audio characteristic matching an audio characteristic associated with an object;
determining (160), based on a video signal from the video capture system, that the object is not present in the field of view, the video signal relating to a time period during which the indicative sound occurred; and
responsive to: detecting the indicative sound; and determining the object is not present in the field of view, determining (170) that the object is present in the monitored environment.

2. The method of claim 1, further comprising:
determining (150) an identifier of the object based on the at least one audio characteristic of the indicative sound.

3. The method of claim 2, wherein determining an identifier of the object comprises:
comparing the at least one audio characteristic with a set of audio identifiers associated with a plurality of different objects, each audio identifier being associated with a respective object and comprising one or more audio characteristics associated with the respective object.

4. The method of claim 1, further comprising:
estimating the location of the object within the monitored environment based on a historical video signal from the video capture system, the historical video signal relating to an earlier time period prior the occurrence of the indicative sound and during which the object was present in the field of view.

5. The method of claim 4, wherein estimating the location of the object within the monitored environment comprises:
analyzing the historical video signal to determining at least one of a location and direction of travel of the object in the field of view; and
extrapolating from the determined location and/or direction of travel of the object in the field of view to estimate the location of the object.

6. The method of claim 1, further comprising
determining (115), using an audio location system, a first location of source of a sound detected by the audio capture system;
determining (120), based on a video signal from the video capture system, a second location of an object present in the field of view;
determining (125) if the first and second locations match; and
responsive to determining that the first and second locations match, associating (130) at least one audio characteristic of the detected sound with the object.

7. The method of claim 6, wherein associating (130) at least one audio characteristic of the detected sound with the object comprises:
analyzing the detected sound to determine one or more audio characteristics of the detected sound;
generating an audio identifier comprising information relating to the determined one or more audio characteristics of the detected sound; and
associating the generated audio identifier with the object.

8. The method of any of claims 1 to 7, wherein the at least one audio characteristic comprises one or more of:
pitch;
loudness;
timbre or tone color;
repetitive pattern;
distinctive sound; and
duration.

9. The method of any of claims 1 to 7, wherein the object comprises a subject and the monitored environment comprises a room of a building.

10. A computer program comprising computer program code means which is adapted, when said computer program is run on a computer, to implement the method of any of claims 1 to 9.

11. A system (200) for detecting the presence of an object (205) in a monitored environment (210), the monitored environment being monitored using an audio capture system (215) adapted to detect sound in the monitored environment and a video capture system (220) having a field of view (FOV) that does not cover the entirety of the monitored environment, the system comprising:
an audio analysis unit (230) configured to detect, based on an audio signal from the audio capture system, an indicative sound having at least one audio characteristic matching an audio characteristic associated with an object;
a video analysis unit (240) configured to determine, based on a video signal from the video capture system, that the object is not present in the field of view, the video signal relating to a time period during which the indicative sound occurred; and
a decision unit (250) configured, responsive to: the audio analysis unit detecting the indicative sound; and the video analysis unit determining the object is not present in the field of view, to determine that the object is present in the monitored environment.

12. The system of claim 11, further comprising:
an identification unit (260) configured to determine an identifier of the object based on the at least one audio characteristic of the indicative sound.

13. The system of claim 11 or 12, further comprising:
an estimation unit (270) configured to estimate the location of the object within the monitored environment based on a historical video signal from the video capture system, the historical video signal relating to an earlier time period prior the occurrence of the indicative sound and during which the object was present in the field of view.

14. The system of any of claims 11 to 13, further comprising
an audio location system (300) configured to determine a first location of source of a sound detected by the audio capture system, and to determine, based on a video signal from the video capture system, a second location of an object present in the field of view.
a comparison unit (310) configured to determine if the first and second locations match; and
an association unit (320) configured, responsive to determining that the first and second locations match, to associate at least one audio characteristic of the detected sound with the object.

15. The system of any of claims 11 to 14, wherein the object comprises a subject and the monitored environment comprises a room of a building.
